(19) **Europäisches Patentamt**
**European Patent Office**
**Office européen des brevets**

(11) **EP 2 263 663 A1**

(12) # EUROPEAN PATENT APPLICATION

(43) Date of publication:
**22.12.2010 Bulletin 2010/51**

(21) Application number: **10006768.5**

(22) Date of filing: **14.11.2005**

(51) Int Cl.:
*A61K 31/12* (2006.01)    *A61K 36/9066* (2006.01)
*A61P 3/10* (2006.01)

(84) Designated Contracting States:
**AT BE BG CH CY CZ DE DK EE ES FI FR GB GR HU IE IS IT LI LT LU LV MC NL PL PT RO SE SI SK TR**

(30) Priority: **13.11.2004 US 988392**

(62) Document number(s) of the earlier application(s) in accordance with Art. 76 EPC:
**05851565.1 / 1 817 941**

(71) Applicant: **Metaproteomics, LLC**
**San Clemente, CA 92673 (US)**

(72) Inventor: **Babish, John G.**
**Brooktondale**
**NY 14817 (US)**

(74) Representative: **Müller-Boré & Partner**
**Patentanwälte**
**Grafinger Straße 2**
**81671 München (DE)**

Remarks:
This application was filed on 30-06-2010 as a divisional application to the application mentioned under INID code 62.

(54) **Compositions comprising curcuminoids and either alpha or beta acids for use in the treatment of diabetes**

(57)    The present invention relates to the use of a first component selected from an alpha acid and a beta acid in the manufacture of a medicament for the treatment of diabetes.

EP 2 263 663 A1

**Description**

This application is a continuation in part of application serial No. 09/885,721, filed June 20, 2001, which is incorporated herein by reference; and is a continuation in part of application serial No. 10/282,236, filed October 25, 2002, which claims the benefit of provisional application serial No. 60/335,062, filed October 26, 2001, now abandoned, which is incorporated herein by reference.

FIELD OF THE INVENTION

The present invention relates generally to a composition comprising a complex mixture of active ingredients exhibiting selective or synergistic inhibition of the expression and/or activity of inducible cyclooxygenase-2 (COX-2) and method for selective inhibition of COX-2 mediated synthesis of prostaglandins. More particularly, the composition comprises mixtures of active ingredients isolated from an extract of hops (*Humulus lupulus*) or as a first component, a curcuminoid species and, as a second component, an active ingredient isolated from an extract of hops (*Humulus lupulus*). The composition functions to inhibit the inducibility and/or activity of inducible cyclooxygenase (COX-2) with little or no significant effect on constitutive cyclooxygenase (COX-1) and can function synergistically.

BACKGROUND OF THE INVENTION

Inflammatory diseases affect more than fifty million Americans. As a result of basic research in molecular and cellular immunology over the last ten to fifteen years, approaches to diagnosing, treating and preventing these immunologically-based diseases has been dramatically altered. One example of this is the discovery of an inducible form of the cyclooxygenase enzyme. Constitutive cyclooxygenase (COX), first purified in 1976 and cloned in 1988, functions in the synthesis of prostaglandins (PGs) from arachidonic acid (AA). Three years after its purification, an inducible enzyme with COX activity was identified and given the name COX-2, while constitutive COX was termed COX-1.

COX-2 gene expression is under the control of pro-inflammatory cytokines and growth factors. Thus, the inference is that COX-2 functions in both inflammation and control of cell growth. While COX-2 is inducible in many tissues, it is present constitutively in the brain and spinal cord, where it may function in nerve transmission for pain and fever. The two isoforms of COX are nearly identical in structure but have important differences in substrate and inhibitor selectivity and in their intracellular locations. Protective PGs, which preserve the integrity of the stomach lining and maintain normal renal function in a compromised kidney, are synthesized by COX-1. On the other hand, PGs synthesized by COX-2 in immune cells are central to the inflammatory process.

The discovery of COX-2 has made possible the design of drugs that reduce inflammation without removing the protective PGs in the stomach and kidney made by COX-1. Combinations of the invention would be useful for, but not limited to, the treatment of inflammation in a subject, and for treatment of other inflammation-associated disorders, such as, as an analgesic in the treatment of pain and headaches, or as an antipyretic for the treatment of fever. For example, combinations of the invention would be useful to treat arthritis, including but not limited to rheumatoid arthritis, spondyloathopathies, gouty arthritis, osteoarthritis, systemic lupus erythematosis, and juvenile arthritis. Such combinations of the invention would be useful in the treatment of asthma, bronchitis, menstrual cramps, tendonitis, bursitis, and skin related conditions such as psoriasis, eczema, bums and dermatitis. Combinations of the invention also would be useful to treat gastrointestinal conditions such as inflammatory bowel disease, Crohn's disease, gastritis, imitable bowel syndrome and ulcerative colitis and for the prevention or treatment of cancer such as colorectal cancer. Compositions of the invention would be useful in treating inflammation in such diseases as vascular diseases, migraine headaches, periarteritis nodosa, thyroiditis, aplastic anemia, Hodgkin's disease, sclerodma, rheumatic fever, type I diabetes, myasthenia gravis, multiple sclerosis, sacoidosis, nephrotic syndrome, Behchet's syndrome, polymyositis, gingivitis, hypersensitivity, swelling occurring after injury, myocardial ischemia and the like.

The compositions of the present invention would also be useful in the treatment of ophthalmic diseases, such as retinopathies, conjunctivitis, uveitis, ocular photophobia, and of acute injury to the eye tissue. The compounds would also be useful in the treatment of pulmonary inflammation, such as that associated with viral infections and cystic fibrosis. The compounds would also be useful for the treatment of certain nervous system disorders such as cortical dementias including Alzheimer's disease. Combinations of the invention are useful as anti-inflammatory agents, such as for the treatment of arthritis, with the additional benefit of having significantly less harmful side effects. As inhibitors of COX-2 mediated biosynthesis of PGE2, these compositions would also be useful in the treatment of allergic rhinitis, respiratory distress syndrome, endotoxin shock syndrome, atherosclerosis, and central nervous system damage resulting from stroke, ischemia and trauma.

Besides being useful for human treatment, these compounds are also useful for treatment of other animals, including horses, dogs, cats, birds, sheep, pigs, etc. An ideal formulation for the treatment of inflammation would inhibit the induction and activity of COX-2 without affecting or with little effect on the activity of COX-1. Historically, the non-steroidal and steroidal anti-inflammatory drugs used for treatment of inflammation lack the specificity of inhibiting COX-2 without affecting COX-1. Therefore, most anti-inflammatory drugs damage the gastrointestinal system when used for extended periods. Thus, new COX-2 specific treatments for inflammation and inflammation-based diseases are urgently needed.

An ideal formulation for the treatment of inflammation would inhibit the induction and activity of COX-2 without affecting the activity of COX-1. However, conventional non-steroidal and steroidal anti-inflammatory drugs lack the specificity of inhibiting COX-2 without affecting COX-1 and are at risk to cause damages on the gastrointestinal system when used for extended periods.

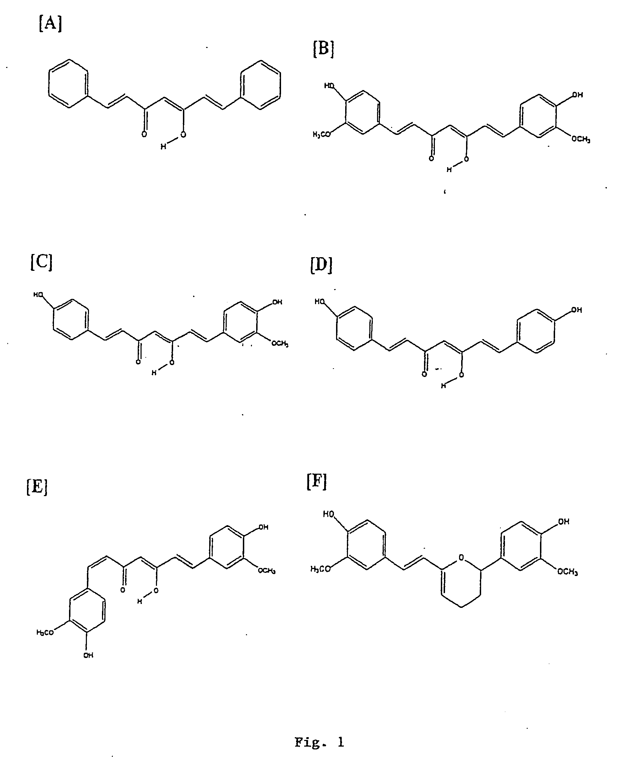

A yellow pigmented fraction isolated from the rhizomes of *Curcuma louga* contains curcuminoids belonging to the dicinnamoyl methane group. Curcuminoids are present to the extent of 3 to 5 percent. They are considered the most important active ingredients and are believed to be responsible for the biological activity of *Curcuma longa.* Though their major activity is anti-inflammatory, curcuminoids have been reported to possess antioxidant, anti-allergic, wound healing, antispasmodic, antibacterial, antifungal and antitumor activity as well. Curcumin (Fig. 1B) was isolated in 1815 and structurally defined in 1910. Other curcuminoids isolated from *Curcum longa* include demethoxycurcumin (Fig. 1C), bisdemethoxycurcumin (Fig. 1D), a cis-trans geometrical isomer of curcumin (Fig. 1E), and cyclocurcumin (Fig 1F). Curcuminoids may be found in other botanicals in addition to *Curcuma longa,* such as *Curcuma xanthorrhiza* and *Curcuma zedoaria.*

Curcuminoids are well known for their anti-inflammatory activity. Tumeric is one of the oldest anti-inflammatory drugs used in Ayurvedic medicine. The anti-inflammatory activity of curcuminoids has been evaluated in inflammatory reaction models such as chemical or physical irritants like carrageenin, cotton pellets, formaldehyde and the granuloma pouch. Human, double-blinded, clinical trials have demonstrated efficacy in rheumatoid arthritis at a dose of 1200 mg curcuminoids/day for five to six weeks. At these doses, however, signs of gastrointestinal (GI) discomfort and stomach irritation are frequently reported.

The GI upset and stomach irritation caused by high doses of curcuminoids may be due to the fact that curcuminoids act on prostaglandin production in a manner similar to that of aspirin and aspirin-like anti-inflammatory agents. Numerous studies have shown that the relative incidence of these GI side effects can be correlated to the relative COX-2 specificity of these agents. The higher the specificity for COX-2 over COX-1, the lower the incidence of GI upset. Thus, aspirin, with a COX-2 specificity of only 0.6, produces a greater incidence of GI distress than curcuminoids, with a reported COX-2 specificity of nearly 3.0. However, the generally accepted COX-2 specificity necessary to significantly reduce the probability of GI upset is 5.0. Thus, combinations of curcuminoids and other compounds or botanical extracts that increase the COX-2 specificity of curcuminoids would provide a novel and improved anti-inflammatory composition.

Hop extraction in one form or another goes back over 150 years to the early nineteenth century when extraction in water and ethanol was first attempted. Even today an ethanol extract is available in Europe, but by far the predominant extracts are organic solvent extracts (hexane) and $CO_2$ extracts (supercritical and liquid). $CO_2$ (typically at 60 bars pressure and 5 to 10°C) is in a liquid state and is a relatively mild, non-polar solvent highly specific for hop soft resins and oils. Beyond the critical point, typically at 300 bars pressure and 60°C, $CO_2$ has the properties of both a gas and a liquid and is a much stronger solvent. The composition of the various extracts is compared in Table 1.

Table 1. Hop Extracts (Percent W/W)

| Component | Hops | Organic Solvent Extract | Super-Critical $CO_2$ | Liquid $CO_2$ |
|---|---|---|---|---|
| Total resins | 12 - 20 | 15 - 60 | 75 - 90 | 70 - 95 |
| Alpha-acids | 2 - 12 | 8 - 45 | 27 - 55 | 30 - 60 |
| Beta-acids | 2 - 10 | 8 - 20 | 23 - 33 | 15 - 45 |
| Essential oils | 0.5 - 1.5 | 0 - 5 | 1 - 5 | 2 - 10 |
| Hard resins | 2 - 4 | 2 - 10 | 5 - 11 | None |
| Tannins | 4 - 10 | 0.5 - 5 | 0.1 - 5 | None |
| Waxes | 1 - 5 | 1 - 20 | 4 - 13 | 0 - 10 |
| Water | 8 - 12 | 1 - 15 | 1 - 7 | 1 - 5 |

At its simplest, hop extraction involves milling, pelleting and re-milling the hops to spread the lupulin, passing a solvent through a packed column to collect the resin components and finally, removal of the solvent to yield a whole or "pure" resin extract.

The main organic extractants are strong solvents and in addition to virtually all the lupulin components, they extract plant pigments, cuticular waxes, water and water-soluble materials.

Supercritical $CO_2$ is more selective than the organic solvents and extracts less of the tannins and waxes and less water and hence water-soluble components. It does extract some of the plant pigments like chlorophyll but less than the organic solvents do. Liquid $CO_2$ is the most selective solvent used commercially for hops and hence produces the most pure whole resin and oil extract. It extracts none of the hard resins or tannins, much lower levels of plant waxes, no plant pigments and less water and water-soluble materials.

As a consequence of this selectivity and the milder solvent properties, the absolute yield of liquid $CO_2$ extract per unit weight of hops is less than when using the other mentioned solvents. Additionally, the yield of alpha acids with liquid $CO_2$ (89-93%) is lower than that of supercritical $CO_2$ (91 - 94%) or the organic solvents (93 - 96%). Following extraction there is the process of solvent removal, which for organic solvents involves heating to cause volatilization. Despite this, trace amounts of solvent do remain in the extract. The removal of $CO_2$, however, simply involves a release of pressure to volatilize the $CO_2$.

The identification of humulone from hops extract as an inhibitor of bone resorption is reported in Tobe, H. et al. 1997. Bone resorption Inhibitors from hop extract. Biosci. Biotech. Biochem 61(1)158-159. Later studies by the same group characterized the mechanism of action of humulone as inhibition of COX-2 gene transcription following TNFalpha stimulation of MC3T3 -E1 cells [Yamamoto, K. 2000. Suppression of cyclooxygenase-2 gene transcription by humulon of bee hop extract studied with reference to glucocorticoid. FEBS Letters 465:103-106].

Thus, it would be useful to identify a natural formulation of compounds that would specifically inhibit or prevent the synthesis of prostaglandins by COX-2 with little or no effect on COX-1. Such a formulation, which would be useful for preserving the health of joint tissues, for treating arthritis or other inflammatory conditions, has not previously been discovered. The term "specific or selective COX-2 inhibitor" embraces compounds or mixtures of compounds that selectively inhibit COX-2 over COX-1. Preferably, the compounds have a median effective concentration for COX-2 inhibition that is minimally five times greater than the media effective concentration for the inhibition of COX-1. For example, if the median inhibitory concentration for COX-2 of a test formulation was 0.2 $\mu$g/mL, the formulation would not be considered COX-2 specific unless the median inhibitory concentration for COX-1 was equal to or greater than 1 $\mu$g/mL.

While glucosamine is generally accepted as being effective and safe for treating osteoarthritis, medical intervention into the treatment of degenerative joint diseases is generally restricted to the alleviation of its acute symptoms. Medical doctors generally utilize non-steroidal and steroidal anti-inflammatory drugs for treatment of osteoarthritis. These drugs, however, are not well adapted for long-term therapy because they not only lack the ability to promote and protect cartilage; they can actually lead to degeneration of cartilage or reduction of its synthesis. Moreover, most non-steroidal, anti-inflammatory drugs damage the gastrointestinal system when used for extended periods. Thus, new treatments for arthritis are urgently needed.

The joint-protective properties of glucosamine would make it an attractive therapeutic agent for osteoarthritis except for two drawbacks: (1) the rate of response to glucosamine treatment is slower than for treatment with anti-inflammatory drugs, and (2) glucosamine may fail to fulfill the expectation of degenerative remission. In studies comparing glucosamine with non-steroidal anti-inflammatory agents, for example, a double-blinded study comparing 1500 mg glucosamine sulfate per day with 1200 mg ibuprofen, demonstrated that pain scores decreased faster during the first two weeks in the ibuprofen patients than in the glucosamine-treated patients. However, the reduction in pain scores continued throughout the trial period in patients receiving glucosamine and the difference between the two groups turned significantly in favor of glucosamine by week eight. Lopes Vaz, A., Double-blind clinical evaluation of the relative efficacy of ibuprofen and glucosamine sulphate in the management of osteoarthritis of the knee in outpatients, 8 Curr. Med Res Opin. 145-149 (1982). Thus, glucosamine may relieve the pain and inflammation of arthritis at a slower rate than the available anti-inflammatory drugs.

An ideal formulation for the normalization of cartilage metabolism or treatment of osteoarthritis would provide adequate chondroprotection with potent anti-inflammatory activity. The optimal dietary supplement for osteoarthritis should enhance the general joint rebuilding qualities offered by glucosamine and attenuate the inflammatory response without introducing any harmful side effects. It should be inexpensively manufactured and comply with all governmental regulations.

However, the currently available glucosamine formulations have not been formulated to optimally attack and alleviate the underlying causes of osteoarthritis and rheumatoid arthritis. Moreover, as with many commercially-available herbal and dietary supplements, the available formulations do not have a history of usage, nor controlled clinical testing, which might ensure their safety and efficacy.

Therefore, it would be useful to identify a composition that would specifically inhibit or prevent the expression of COX-2 enzymatic activity, while having little or no effect on COX-1 metabolism so that these could be used at sufficiently low doses or at current clinical doses with no adverse side effects.

SUMMARY OF THE INVENTION

The present invention provides a composition comprising an effective amount of component I selected from the group consisting of alpha acids and beta acids and an effective amount of at least one component II selected from the group consisting of alpha acids, beta acids, essential oils, fats and waxes, with the proviso that component I and II are not the same compound. Preferably, the composition comprises two or more active ingredients selected from the group consisting of α-acid, β-acid and essential oil. The active ingredients of the present invention are preferably made from hops extract. The composition functions synergistically to inhibit the activity of inducible COX-2 with little or no effect on COX-1.

The present invention also provides a composition comprising, as a first component, a curcuminoid species and a second compound that would specifically and synergistically enhance the anti-inflammatory effect of the curcuminoid. The composition comprises a curcuminoid species and at least one member selected from the group consisting of an alpha-acid, and a beta-acid or derivatives thereof. Any curcuminoid, alpha-acid or beta-acid species is inclusive of derivatives of the respective genus. However, additional species or mixtures of species within the various genera may be present in the composition, which is limited in scope only by the combinations of species within the various genera that exhibit the claimed synergistic functionality. The composition functions synergistically to inhibit the inducibility and/or activity of COX-2 with little or no effect on COX-1.

The present invention further provides a composition of matter that enhances the function of or increases the rate at which glucosamine or chondrotin sulfate normalize joint movement or reduce the symptoms of osteoarthritis.

One specific embodiment of the present invention is a composition comprising a 30 to 60 weight percent of α-acid, 15 to 45 weight percent of β-acid and 3 to 6 weight percent of essential oil. The composition optionally comprises 2 to 8 weight percent of fats and waxes. Preferably, the α-acid, β-acid, essential oil, fats or waxes are from a hops extract, which is preferably prepared by $CO_2$ extraction.

Another specific embodiment of the present invention is a composition comprising an effective amount of curcumin and at least one compound selected from the group consisting of humulone and lupulone.

The present invention further provides a method of dietary supplementation and a method of treating inflammation or inflammation-based diseases in an animal which comprises providing to the animal suffering symptoms of inflammation, including pain and swelling, the composition of the present invention containing two or more active ingredients selected from the group consisting of α-acid, β-acid and essential oil and continuing to administer such a dietary supplementation of the composition until said symptoms are eliminated or reduced.

The present invention also provides a method of dietary supplementation and a method of treating inflammation or inflammation-based diseases in an animal which comprises providing to the animal suffering symptoms of inflammation the composition of the present invention containing a second component which specifically and synergistically enhances the anti-inflammatory effect of curcuminoids and continuing to administer such a dietary supplementation of the composition until said symptoms are eliminated or reduced:

BRIEF DESCRIPTION OF THE DRAWINGS

FIG. 1 illustrates the general chemical structure of [A] the curcuminoid genus and [B], [C], [D], [E] and [F], respectively, as curcumin, demethoxycurcumin bisdemethoxycurcumin, the cis-trans geometrical isomer of curcumin, and cyclocurcumin as species within that genus.

FIG. 2, [A] and [B] respectively, illustrate the general chemical structures of the alpha-acid genus and humulone as a species within that genus.

FIG. 3, [A] and [B], respectively, illustrates the general chemical structures of the beta-acid genus and lupulone.

DETAILED DESCRIPTION OF THE INVENTION

Before the present composition and methods of making and using thereof are disclosed and described, it is to be understood that this invention is not limited to the particular configurations, as process steps, and materials may vary somewhat. It is also intended to be understood that the terminology employed herein is used for the purpose of describing particular embodiments only and is not intended to be limiting since the scope of the present invention will be limited only by the appended claims and equivalents thereof.

It must be noted that, as used in this specification and the appended claims, the singular forms "a," "an," and "the" include plural referents unless the context clearly dictates otherwise.

The present invention provides a composition having a selective inhibitory effect on the activity of COX-2, said composition comprising an effective amount of component I selected from the group consisting of alpha acids and beta acids and an effective amount of at least one component II selected from the group consisting of alpha acids, beta acids, essential oils, fats and waxes, with the proviso that component 1 and II are not the same compound. More particularly, the composition comprises two or more active ingredients selected from the groups consisting of α-acids, β-acids and essential oils. Preferably, the active ingredients of the present invention are made from Hops extract. Preferably, composition comprising an 30 to 60 weight percent of α-acids, 15 to 45 weight percent of β-acids and 3 to 6 weight percent of essential oils. The composition optionally comprises 2 to 8 weight percent of fats and waxes. Preferably, the α-acids, β-acids, essential oils, fats or waxes are from a hop extract, which is preferably prepared by $CO_2$ extraction. The composition provided by the present invention can be formulated as a dietary supplement or therapeutic composition. The composition functions to inhibit the inducibility and/or activity of COX-2 with little or no effect on COX-1.

The present also invention provides a composition having a synergistic inhibitory effect on the expression and/or activity of COX-2. More particularly, the composition comprises, as a first component, an active curcuminoid and, as a second component, at least one member selected from the group consisting of an active alpha-acid or active beta-acid or derivatives thereof as more specifically described above. The composition provided by the present invention can be formulated as a dietary supplement or therapeutic composition. The composition functions synergistically to inhibit the inducibility and/or activity of COX-2 with no significant effect on COX-1.

As used herein, the term "dietary supplement" refers to compositions consumed to affect structural or functional changes in physiology. The term "therapeutic composition" refers to any compounds administered to treat or prevent a disease.

As used herein, the term "COX inhibitor" refers to a composition of natural compounds that is capable of inhibiting the activity or expression of COX-2 enzymes or is capable of inhibiting or reducing the severity, including pain and swelling, of a severe inflammatory response.

As used herein, the term "active curcuminoid" refers to a species within the curcuminoid genera that is capable of inhibiting the inducibility and/or activity of COX-2 while having little or no effect on COX-1 or is capable of inhibiting or reducing the severity of a severe inflammatory response. The preferred "active curcuminoid" is curcumin.

As used herein, the term "hop extract " refers to the solid material resulting from (1) exposing a hops plant product to a solvent, (2) separating the solvent from the hops plant product, and (3) eliminating the solvent.

As used herein, the term "solvent" refers to a liquid of aqueous or organic nature possessing the necessary characteristics to extract solid material from the hop plant product. Examples of solvents would include water, steam, superheated water, methanol, ethanol, hexane, chloroform, liquid $CO_2$, liquid $N_2$ or any combinations of such materials.

As used herein, the term "$CO_2$ extract" refers to the solid material resulting from exposing a hops plant product to a liquid or supercritical $CO_2$ preparation followed by the removal of the $CO_2$.

As used herein, the term "α-acid fraction" refers to compounds isolated from hops plant products including, among others, humulone, cohumulone, isohumulone, isoprehumulone, hulupone, adhumulone, xanthohumol A and xanthohumol B.

As used herein, the term "β-acid fraction" refers to compounds collectively known as lupulones including among others lupulone, colupulone, adlupulone, tetrahydroisohumulone, and hexahydrocolupulone,

As used herein, the term "essential oil fraction" refers to a complex mixture of components consisting chiefly of myrcene, humulene, beta-caryophyleen, undecane-2-on, and 2-methyl-but-3-en-ol.

As used herein, the term "fats " refers to triacylglyerol esters of fatty acids.

As used herein, the term "waxes " refers to triacylglycerol ethers or esters of extremely long chain (>25 carbons) fatty alcohols or acids.

Therefore, one preferred embodiment of the present invention is a composition comprising a combination of an effective amount of two or more active ingredients selected from the group consisting of α-acid, β-acid and essential oil. The composition of the present invention functions to specifically inhibit the inducibility and/or activity of COX-2 while showing little or no effect on COX-1. Therefore, the composition of the present invention essentially eliminates the inflammatory response, including pain and swelling, rapidly without introducing any harmful side effects.

As used herein, the term "active curcuminoid", "active ingredient of hop extract" or derivatives thereof refers to naturally occurring or synthetic derivatives of species within the scope of the respective genera that are capable of inhibiting the inducibility and/or activity of COX-2 while having little or no effect on COX-1 or are capable of inhibiting or reducing the severity of an inflammatory response. Representative species within each genus are listed in Table 2. Of the species listed under each genus in Table 2, those containing at least one asterisk (*) are preferred and those containing two asterisks (**) are particularly preferred.

**TABLE 2**

| CURCUMINOIDS | ALPHA-ACIDS | BETA-ACIDS |
|---|---|---|
| Curcumin** | Humulone** | Lupulone** |
| Demethoxycurcumin** | Cohumulone* | Colupulone* |
| Bisdemethoxycurcumin** | Isohumulone* | Adlupulone* |

(continued)

| CURCUMINOIDS | ALPHA-ACIDS | BETA-ACIDS |
|---|---|---|
| Cis-trans curcumin* | Isoprehumulone* | Tetrahydroisohumulone* |
| Cyclocurcumin* | Hulupone* | Hexahydrocolupulone* |
| | Adhumulone* | Diydro-isohumulone* |
| | Xanthohumulone A* | |
| | Xanthohumulone B* | |

"Conjugates" of curcuminoids, alpha- and beta-acids or derivatives thereof means curcuminoids, alpha-acids, and beta-acids covalently bound or conjugated to a member selected from the group consisting of mono- or di- saccharides, amino acids, sulfates, succinate, acetate and glutathione. Preferably, the mono- or di- saccharide is a member selected from the group consisting of glucose, mannose, ribose, galactose, rhamnose, arabinose, maltose, and fructose.

Therefore, one preferred embodiment of the present invention is a composition comprising effective amount of curcumin, as a first component, and a second component selected from the group consisting of alpha-acids and beta-acids. The resulting formulation of these combinations functions to synergistically inhibit the inducibility and/or activity of COX-2 while showing little or no effect on COX-1. Therefore, the composition of the present invention essentially eliminates the inflammatory response rapidly without introducing any harmful side effects.

Preferably, the curcuminoid genus, as represented by FIG. 1[A] and specifically exemplified by curcumin in FIG.1 [B] is a pharmaceutical grade botanical extract such as can be obtained commercially, for example, from Sabinsa, 121 Ethel Road West, Piscataway, NJ. Other curcuminoids that may be employed include demethoxycurcumin (FIG 1 [C]) bisdemethoxycurcumin (FIG. 1[D]), a cis-trans curcumin (Fig 1E) and cyclocurcumin (FIG 1F). The curcuminoid used can be readily obtained from *Curcuma longa* L. Pharmaceutical grade curcuminoid extract is standardized to have a curcuminoid content of greater than 70 percent. The pharmaceutical botanical grade extract must pass extensive safety and efficacy procedures. As employed in the practice of the present invention, the extract has a curcuminoid content of about 1 to 99 percent by weight. Preferably, the minimum curcumin content is about 70 percent by weight. Alternatively, the curcumin may be synthesized using standard techniques known in chemical synthesis.

The essence of the present invention is that, rather than modifying the curcuminoid molecule to achieve greater efficacy and lower toxicity, a second component is added that acts in a synergistic manner. Therefore, this invention relates to the discovery that when combining a curcuminoid with a second molecule, selected from the group consisting of a alpha-acids or a beta-acids and derivatives thereof, the combination produces a synergistic effect in the target cell. One such synergistic response would be the specific inhibition of inducible COX-2. Preferably, the second molecule is a member selected from the group consisting of humulone and lupulone.

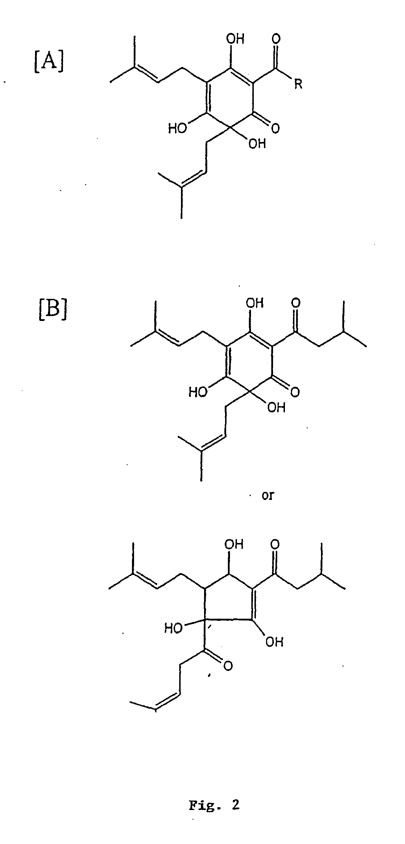

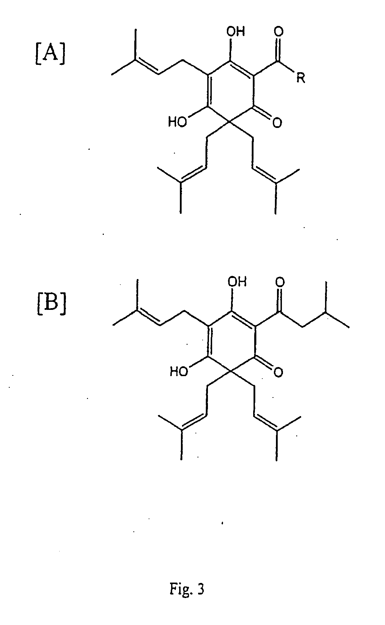

Preferably, the alpha-acid genus, as represented by FIG. 2 [A] and specifically by humulone in FIG. 2 [B], and the beta-acid genus, as represented by FIG. 3 [A] and specifically exemplified by lupulone (FIG. 2 [B]) is a pharmaceutical grade preparation such as can be obtained commercially, for example, from Hopunion. (Yakima, WA).

Without limiting the invention, the inhibition of the activity of the COX-2 enzyme by alpha-acids or beta-acids provides a dual, synergistic effect with curcuminoids. Thus, the second compound selected from the group consisting of alpha-acids and beta-acids increases the anti-inflammatory activity of the curcininoids. The result of the combinations of this invention is a more selective effect on the activity of COX-2 at lower doses of curcuminoids than would normally be required. By decreasing the dose of curcuminoids to achieve the desired COX-2 inhibition, the probability of side effects from this compound decreases almost exponentially. The second compound selected from the group consisting of alpha-acids and beta-acids can provide hepatoprotection, antitumor promotion, antihyperlipidermia, antihyperglycermia and protection against ulcer formation from COX-1 inhibition by the curcuminoids.

The pharmaceutical grade extract must pass extensive safety and efficacy procedures. Pharmaceutical grade $CO_2$ hops extract refers to a preparation wherein the concentration of hops extract, as employed in the practice of the invention, has an $\alpha$-acid content of about 10 to 95 percent by weight. Preferably, the $\alpha$-acid content is greater than 45 percent by weight. The range of $\beta$-acid content in a pharmaceutical grade hops extract is about 10 to 95 percent by weight. Preferably, the $\beta$-acid content is greater than 45 percent by weight. The pharmaceutical grade extracts are particularly preferred. A daily dose (mg/kg-day) of the present dietary supplement would be formulated to deliver, about 0.001 to 100 mg $CO_2$ extract of hops extract per kg body weight of the animal. In another embodiment, preferably, a daily dose (mg/kg-day) of the present dietary supplement would be formulated to deliver, per kg body weight of the animal, about 0.001 to 30.0 mg curcuminoids, and about 0.5 to 20.0 mg alpha-acids or beta-acids.

The composition of the present invention for topical application would contain about 0.001 to 10 wt%, preferably 0.01 to 1 wt% of pharmaceutical grade $CO_2$ hops extract. In another embodiment, the composition of the present invention

EP 2 263 663 A1

for topical application would contain one of the following: about 0.001 to 1 wt%, preferably 0.01 to 1 wt% curcuminoids, and about 0.025 to 1 wt%, preferably 0.05 to 1 wt% alpha-acids or beta-acids.

The preferred composition of the present invention would produce serum or target tissue concentrations of any of the $\alpha$-acid or $\beta$-acid components in the range of about 0.005 to 10,000 ng/mL. In another embodiment, the preferred composition of the present invention would produce serum concentrations in the following range: 0.0001 to 10 $\mu$M of curcuminoids, and 0.001 to 10 $\mu$M alpha-acids or beta-acids.

TABLE 3 below provides a list of diseases in which COX-2 enzyme expression and activity may play a significant role and therefore are appropriate targets for normalization or treatment by the invention.

**TABLE 3**

| DISEASE | TISSUE |
|---|---|
| Addison's Disease | Adrenal |
| Allergies | Inflammatory cells |
| Alzheimer Disease | Nerve calls |
| Arthritis | Inflammatory cells |
| Atherosclerosis | Vessel wall |
| Colon Cancer | Intestine |
| Crohn's Disease | Intestine |
| Diabetes (type 1)/type II | Pancreas |
| Eczema | Skin/Inflammatory cells |
| Graves Disease | Thyroid |
| Guillain-Barre Syndrome | Nerve cells |
| Inflammatory Bowel Disease | Intestine |
| Leukemia | Immune cells |
| Lymphomas | Immune cells |
| Multiple Sclerosis | Nerve cells |
| Myasthenia Gravis | Neuromuscular junction |
| Ostcoarthrilis | Joint lining |
| Psoriasis | Skin |
| Primary Biliary Cirrhosis | Liver |
| Rheumatoid Arthritis | Joint lining |
| Solid Tumors | Various |
| Systemic Lupus Erythemalosis | Multiple tissues |
| Uvcitis | Eye |

In addition to the combination of component I selected from the group consisting of alpha acids and beta acids and at least one component II selected from the group consisting of alpha acids, beta acids, essential oils, fats and waxes, with the proviso that component I and II are not the same compound, and the combination of curcuminoids and alpha-acids, beta-acids or derivatives, the present composition for dietary application may include various additives such as other natural components of intermediary metabolism, vitamins and minerals, as well as inert ingredients such as talc and magnesium stearate that are standard excipients in the manufacture of tablets and capsules.

As used herein, "pharmaceutically acceptable carrier" includes any and all solvents, dispersion media, coatings, isotonic and absorption delaying agents, sweeteners and the like. These pharmaceutically acceptable carriers may be prepared from a wide range of materials including, but not limited to, diluents, binders and adhesives, lubricants, disintegrants, coloring agents, bulking agents, flavoring agents, sweetening agents and miscellaneous materials such as buffers and absorbents that may be needed in order to prepare a particular therapeutic composition. The use of such media and agents for pharmaceutically active substances is well known in the art. Except insofar as any conventional media or agent is incompatible with the active ingredients, its use in the present composition is contemplated. In one embodiment, talc and magnesium stearate are included in the present formulation. When these components are added they are preferably, Astac Brand 400 USP talc powder and the veritable grade of magnesium stearate. Other ingredients known to affect the manufacture of this composition as a dietary bar or functional food can include flavorings, sugars, amino-sugars, proteins and/or modified starches, as well as fats and oils.

The dietary supplements, lotions or therapeutic compositions of the present invention can be formulated in any manner known by one of skill in the art. In one embodiment, the composition is formulated into a capsule or tablet using techniques available to one of skill in the art. In capsule or tablet form, the recommended daily dose for an adult human or animal would preferably be contained in one to six capsules or tablets. However, the present compositions may also be formulated in other convenient forms, such as an injectable solution or suspension, a spray solution or suspension, a lotion, gum, lozenge, food or snack item. Food, snack, gum or lozenge items can include any ingestible ingredient, including sweeteners, flavorings, oils, starches, proteins, fruits or fruit extracts, vegetables or vegetable extracts, grains, animal fats or proteins. Thus, the present compositions can be formulated into cereals, snack items such as chips, bars, chewable candies or slowly dissolving lozenges.

The present invention contemplates treatment of all types of inflammation-based diseases, both acute and chronic. The present formulation reduces the inflammatory response and thereby promotes healing of, or prevents further damage to, the affected tissue. A pharmaceutically acceptable carrier may also be used in the present compositions and formulations.

According to the present invention, the animal may be a member selected from the group consisting of humans, non-human primates, such as dogs, cats, birds, horses, ruminants or other warm blooded animals. The invention is directed primarily to the treatment of human beings. Administration can be by any method available to the skilled artisan, for example, by oral, topical, transdermal, transmucosal, or parenteral routes.

The following examples are intended to illustrate but not in any way limit the invention.

EXAMPLE I

Selective Inhibition of Cyclooxygenase-2 Mediated Prostaglandin E2 by a $CO_2$ Extract of Hops

This example illustrates a superior COX-2 selectivity of the $CO_2$ hops extract of the present invention compared to the pure compound humulone described in the prior art. Therefore it is to be inferred that the effectiveness of the $CO_2$ hops extract of the present invention would be superior to the pure compound humulone described in the prior art.

**Inhibition of COX-2 Mediated Production of PGE2 by $CO_2$ extract of Hops** - *Equipment* - balancer, analytical, Ohaus Explorer (Ohaus Model #EO1140, Switzerland), biosafety cabinet (Forma Model #F1214, Marietta, Ohio), pipettor, 100 to 1000 $\mu$L (VWR Catalog #4000-208, Rochester, NY), cell hand tally counter (VWR Catalog #23609-102, Rochester, NY), $CO_2$ incubator (Forma Model #F3210, Marietta, Ohio), hemacytometer (Hausser Model #1492, Horsham, PA), microscope, inverted (Leica Model #DM IL, Wetzlar, Germany), multichannel pipettor, 12-Channel (VWR Catalog #53501-662, Rochester, NY), Pipet Aid (VWR Catalog #53498-103, Rochester, NY), Pipettor, 0.5 to 10 $\mu$L (VWR Catalog #4000-200, Rochester, NY), pipettor, 100 to 1000 $\mu$L (VWR Catalog #4000-208, Rochester, NY), pipettor, 2 to 20 $\mu$L (VWR Catalog #4000-202, Rochester, NY), pipettor, 20 to 200 $\mu$L (VWR Catalog #4000-204, Rochester, NY), PURELAB Plus Water Polishing System (U.S. Filter, Lowell, MA), refrigerator, 4°C (Forma Model #F3775, Marietta, Ohio), vortex mixer (VWR Catalog #33994-306, Rochester, NY), water bath (Shel Lab Model #1203, Cornelius, OR).

*Cells, Chemicals, Reagents and Buffers* - Cell scrapers (Corning Catalog #3008, Corning, NY), dimethylsulfoxide (DMSO) (VWR Catalog #5507, Rochester, NY), Dulbecco's Modification of Eagle's Medium (DMEM) (Mediatech Catalog #10-013-CV, Herndon, VA), fetal bovine serum, heat inactivated (FBS-H1) (Mediatech Catalog #35-011-CV, Herndon, VA), lipopolysaccharide (LPS)(Sigma Catalog #L-2654, St. Louis, MO), microfuge tubes, 1.7 mL (VWR Catalog #20172-698, Rochester, NY), penicillin/streptomycin (Mediatech Catalog #30-001-CI, Herndon, VA), pipet tips for 0.5 to 10 $\mu$L pipettor (VWR Catolog #53509-138, Rochester, NY), pipet tips for 100-1000 $\mu$L pipettor (VWR Catolog #53512-294, Rochester, NY), pipet tips for 2-20 $\mu$L and 20-200 $\mu$L pipettors (VWR Catolog #53512-260, Rochester, NY), pipets, 10 mL (Becton Dickinson Catalog #7551, Marietta, OH), pipets, 2 mL (Becton Dickinson Catalog #7507, Marietta, OH, pipets, 5 mL (Becton Dickinson Catalog #7543, Marietta, OH), RAW 264.7 Cells (American Type Culture Collection Catalog #TIB-71, Manassas, VA), test compounds (liquid $CO_2$ hops extract from Hopunion, Yakima, WA), tissue culture plates, 96-well (Becton Dickinson Catalog #3075, Franklin Lanes, NJ), Ultra-pure water (Resistance =18 megaOhm-cm deionized water).

*General Procedure* - RAW 264.7 cells, obtained from ATCC, were grown in DMEM medium and maintained in log phase. The DMEM growth medium was made as follows : 50 mL of heat inactivated FBS and 5 mL of penicillin/streptomycin was added to a 500 mL bottle of DMEM and stored at 4°C. For best result the medium is to be used within three months and warmed to 37°C in water bath before use.

On day one of the experiment, the log phase 264.7 cells were plated at $8 \times 10^4$ cells per well in 0.2 mL growth medium per well in a 96-well tissue culture plate in the morning. At the end of the day 1 (6 to 8 hours post plating), 100 $\mu$L of growth medium from each well were removed and replaced with 100 $\mu$L fresh medium. A 1.0 mg/mL solution of LPS, which is used to induce the expression of COX-2 in the RAW 264.7 cells, was prepared by dissolving 1.0 mg of LPS in 1 mL DMSO. It was vortexed until it dissolved and was stored at 4°C. Melt at room temperature or in a 37°C water bath before use. Make up a new solution every 60 days.

On day two of the experiment, liquid $CO_2$ hops extract was prepared as 1000X stock in DMSO. For example, if the final concentration of the test material is to be 10 $\mu$g/mL, a 10 mg/mL stock should be prepared by dissolving 10 mg of the test material in 1 mL of DMSO. For the best result, fresh liquid $CO_2$ hops extract should be prepared on the day of the experiment. In 1.7 mL microfuge tubes, 1 mL DMEM without FBS was added for test concentrations of 0.05, 0.10, 0.5, and 1.0 $\mu$g/mL. 2 $\mu$L of the 1000X DMSO stock of the test material was added to the 1 mL of medium without FBS. The tube contained the final concentration of the test material concentrated 2-fold and then placed in an incubator for 10 minutes to equilibrate.

One-hundred microliters of medium was removed from each well of the cell plates prepared on day one. One-hundred microliter of equilibrated 2X final concentration the test compounds was added to cells and incubated for 90 minutes. LPS in DMEM without FBS was prepared by adding 44 $\mu$L of the 1 mg/mL DMSO stock to 10 mL of medium. For each well of cells to be stimulated, 20 $\mu$L of LPS (final concentration of LPS is 0.4 $\mu$g/mL of LPS) was added and incubated for 24 hours.

On day 3, the appearance of the cells was observed. One-hundred microliter supernatent medium from each well was transferred to a clean microfuge tube for the determination of amount of PGE2 in the medium.

<u>Determination of COX-1 Enzyme Inhibition by Hops Extract</u> - The ability of a test material to inhibit COX-1 synthesis of PGE2 was determined essentially as described by Noreen, Y., et al. (J. Nat. Prod. 61, 2-7, 1998).

*Equipment -* balancer (2400 g, Acculab VI-2400, VWR Catalog #11237-300, Rochester, NY), balancer, analytical, Ohaus Explorer (Ohaus Model #EO1140, Switzerland), biosafety cabinet (Forma Model #F1214, Marietta, Ohio), Freezer, -30°C (Forma Model #F3797), Freezer, -80°C Ultralow (Forma Model #F8516, Marietta, OH), heated stirring plate (VWR Catalog #33918-262, Rochester, NY), ice maker (Scotsman Model #AFE400A-1A, Fairfax, SC), multichannel pipettor, 12-Channel (VWR Catalog #53501-662, Rochester, NY), Multichannel Pipettor, 8-Channel (VWR Catalog #53501-660, Rochester, NY), orbital shaker platform (Scienceware #F37041-0000, Pequannock, NJ), pH meter (VWR Catalog #33221-010, Rochester, NY), pipet aid (VWR Catalog #53498-103, Rochester, NY), pipettor, 0.5 to 10 $\mu$L (VWR Catalog #4000-200, Rochester, NY), pipettor, 100 to 1000 $\mu$L (VWR Catalog #4000-208, Rochester, NY), pipettor, 2 to 20 $\mu$L (VWR Catalog #4000-202, Rochester, NY), pipettor, 20 to 200 $\mu$L (VWR Catalog #4000-204, Rochester, NY), PURELAB Plus Water Polishing System (U.S. Filter, Lowell, MA), refrigerator, 4°C (Forma Model #F3775, Marietta, Ohio), vacuum chamber (Sigma Catalog #Z35, 407-4, St. Louis, MO), vortex mixer (VWR Catalog #33994-306, Rochester, NY)

*Supplies and Reagents* - 96-Well, round-bottom plate (Nalge Nunc #267245, Rochester, NY), arachidonic acid (Sigma Catalog #A-3925, St. Louis, MO), centrifuge tubes, 15 mL, conical, sterile (VWR Catalog #20171-008, Rochester, NY), COX-1 enzyme (ovine) 40,000 units/mg (Cayman Chemical Catalog #60100, Ann Arbor, MI), dimethylsulfoxide (DMSO) (VWR Catalog #5507, Rochester, NY), ethanol 100% (VWR Catalog #MK701908, Rochester, NY), epinephrine (Sigma Catalog #E-4250, St. Louis, MO), glutathione (reduced) (Sigma Catalog # G-6529, St. Louis, MO), graduated cylinder, 1000 mL (VWR Catalog #24711-364, Rochester, NY), hematin (porcine) (Sigma catalog# H-3281, St. Louis, MO), hydrochloric acid (HCl) (VWR Catalog #VW3110-3, Rochester, NY), KimWipes (Kimberly Clark Catalog #34256, Roswell, GA), microfuge tubes, 1.7 mL (VWR Catalog #20172-698, Rochester, NY), NaOH (Sigma Catalog #S-5881, St. Louis, MO), pipet tips for 0.5 to 10 $\mu$L pipettor (VWR Catolog #53509-138, Rochester, NY), pipet tips for 100-1000 $\mu$L pipettor (VWR Catolog #53512-294, Rochester, NY), pipet tips for 2-20 $\mu$L and 20-200 $\mu$L pipettors (VWR Catolog #53512-260, Rochester, NY), prostaglandin E2 (Sigma Catalog # P-5640, St. Louis, MO), prostaglandin F2alpha (Sigma Catalog #P-0424, St. Louis, MO), stir bar, magnetic (VWR Catalog #58948-193, Rochester, NY), storage bottle, 1000 mL (Coming Catalog #1395-1 L, Corning, NY), storage bottle, 100 mL (Coming Catalog #1395-100, Corning, NY), $CO_2$ extract of hops (Hopunion, Yakima, WA), Tris-HCl (Sigma Catalog #T-5941, St. Louis, MO), ultra-pure water (Resistance =18 megaOhm-cm deionized water).

*General Procedure -* Oxygen-free 1.0M Tris-HCl buffer (pH 8.0) was prepared as follows: In a 1000 mL beaker, 12.11g Trizma HCl was dissolved into 900 mL ultra-pure water. The beaker was placed on a stir plate with a stir bar. NaOH was added until the pH reached 8.0. The volume was adjusted to a final volume of 1000mL and stored in a 1000 mL storage bottle.

The Tris-HCl buffer was placed into a vacuum chamber with a loose top and the air pump was turned on until the buffer stopped bubbling. The vacuum chamber was turned off and the storage bottle was covered tight. This step was repeated each time when the oxygen-free Tris-HCl buffer was used.

One mL cofactor solution was prepared by adding 1.3 mg (-) epinephrine, 0.3 mg reduced glutathione and 1.3 mg hematin to 1 mL oxygen free Tris-HCl buffer. Solutions of the test material were prepared as needed. i.e. 10 mg of aspirin was weighed and dissolved into 1 mL DMSO.

Enzyme was dissolved in oxygen free Tris-HCl buffer as follows, i.e. on ice, 6.5 $\mu$L of enzyme at 40,000 units/mL was taken and added to 643.5 $\mu$L of oxygen free Tris-HCl buffer. This enzyme solution is enough for 60 reactions. The COX-1 enzyme solution was prepared as follows. In a 15 mL centrifuge tube, 10 $\mu$L COX-1 enzyme at 40,000 units/mL was added in oxygen free Tris-HCl with 50 $\mu$L of the cofactor solution per reaction. The mixture was incubated on ice for 5 minutes (i.e. for 60 reactions add 650 $\mu$l enzyme in oxygen free Tris-HCl buffer with 3.25 mL cofactor solution).

Sixty $\mu$l of the enzyme solution was combined with 20 $\mu$l of the test solution in each well of a 96 well plate. Final concentrations of the test solutions were 100, 50, 25, 12.5, 6.25 and 3.12 $\mu$g/mL. The plates were preincubated on ice for 10 minutes. 20 $\mu$L arachidonic acid (30$\mu$M) was added and incubated for 15 minutes at 37°C.

Two M HCl was prepared by diluting 12.1 N HCl. In a 100 mL storage bottle, 83.5 mL ultra-pure water was added and then 16.5 mL 12.1 N HCl was added. It was stored in a 100 mL storage bottle and placed in the biosa fty cabinet (always add acid last). The reaction was terminated by adding 10 $\mu$L 2 M HCl. The final solution was used as the supernate for the PGE$_2$ assay.

__Determination of PGE2 Concentration in Medium__ - The procedure followed was that essentially described by Hamberg, M. and Samuelsson, B. (J. Biol. Chem. 1971. 246, 6713-6721); however a commercial, nonradioactive procedure was employed.

*Equipment* - freezer, -30°C (Forma Model #F3797), heated stirring plate (VWR Catalog #33918-262, Rochester, NY), multichannel pipettor, 12-Channel (VWR Catalog #53501-662, Rochester, NY), orbital shaker platform (Scienceware #F37041-0000, Pequannock, NJ), Pipet Aid (VWR Catalog #53498-103, Rochester, NY), pipettor, 0.5 to 10 $\mu$L (VWR Catalog #4000-200, Rochester, NY), pipettor, 100 to 1000 $\mu$L (VWR Catalog #4000-208, Rochester, NY), pipettor, 2 to 20 $\mu$L (VWR Catalog #4000-202, Rochester, NY), pipettor, 20 to 200 $\mu$L (VWR Catalog #4000-204, Rochester, NY), plate reader (Bio-tek Instruments Model #Elx800, Winooski, VT), PURELAB Plus Water Polishing System (U.S. Filter, Lowell, MA), refrigerator, 4°C (Forma Model #F3775, Marietta, Ohio).

*Chemicals, Reagents and Buffers* - Prostaglandin E$_2$ EIA Kit-Monoclonal 480-well (Cayman Chemical Catalog # 514010, Ann Arbor, MI), centrifuge tube, 50 mL, conical, sterile (VWR Catalog #20171-178, Rochester, NY), Dulbecco's Modification of Eagle's Medium (DMEM) (Mediatech Catalog #10-013-CV, Herndon, VA), graduated cylinder, 100 mL(VWR Catalog #24711-310, Rochester, NY), KimWipes (Kimberly Clark Catalog #34256, Roswell, GA), microfuge tubes, 1.7 mL (VWR Catalog #20172-698, Rochester, NY), penicillin/streptomycin (Mediatech Catalog #30-001-Cl, Herndon, VA), pipet tips for 0.5 to 10 $\mu$L pipettor (VWR Catolog #53509-138, Rochester, NY), pipet tips for 100-1000 $\mu$L pipettor (VWR Catolog #53512-294, Rochester, NY), pipet tips for 2-20 $\mu$L and 20-200 $\mu$L pipettors (VWR Catolog #53512-260, Rochester, NY), pipets, 25 mL (Becton Dickinson Catalog #7551, Marietta, OH), storage bottle, 100 mL (Coming Catalog #1395-100, Corning, NY), storage bottle, 1000 mL (Coming Catalog #1395-1L, Corning, NY), ultra-pure water (Resistance =18 megaOhm-cm deionized water).

*General Procedure* - EIA Buffer was prepared by diluting the contents of EIA Buffer Concentrate (vial #4) with 90ml of Ultra-pure water. The vial #4 was rinsed several times to ensure all crystals had been removed and was placed into a 100 mL storage bottle and stored at 4°C.

The Wash Buffer was prepared by diluting Wash Buffer Concentrate (vial #5) 1:400 with Ultra-pure water. 0.5 ml/liter of Tween 20 (vial #5a) was then added (using a syringe for accurate measurement). i.e. (For one liter Wash Buffer add 2.5ml Wash Buffer Concentrate, 0.5ml Tween-20, and 997ml Ultra-pure water.) The solution was stored in a I liter storage bottle at 4°C.

The Prostaglandin E$_2$ standard was reconstituted as follows. A 200$\mu$L pipet tip was equilibrated by repeatedly filling and expelling the tip several times in ethanol. The tip was used to transfer 100 $\mu$L of the PGE$_2$ Standard (vial #3) into a 1.7 mL microfuge tube. 900$\mu$l Ultra-pure water was added to the tube and stored at 4°C, which was stable for ~6 weeks.

The Prostaglandin E$_2$ acetylcholinesterase tracer was reconstituted as follows. 100 $\mu$L PGE$_2$ tracer (vial #2) was taken and mixed with 30 mL of the EIA Buffer in a 50 mL centrifuge tube and stored at 4°C. The solution should be used within five weeks.

The Prostaglandin E$_2$ monoclonal antibody was reconstituted as follows. 100$\mu$L PGE$_2$ Antibody (vial #1) was taken and mixed with 30 mL of the EIA buffer in a 50 mL centrifuge tube and stored at 4°C. This solution should be used up within 5 weeks.

DMEM with penicillin/streptomycin was prepared by adding 5 mL penicillin/streptomycin into 500 mL DMEM and stored at 4°C.

The plate was set up as follows: Each plate contained a minimum of two blanks (B), two non-specific binding wells (NSB), two maximum binding wells (B$_0$), and an eight point standard curve run in duplicate (S1-S8). Each sample was assayed at a minimum of two dilutions and each dilution was run in duplicate.

The standard was prepared as follows: Eight 1.7 mL microuge tubes were labeled as tube 1-8. 900 $\mu$L DMEM into was put in tube 1 and 500 $\mu$L DMEM into tubes 2-8. 100 $\mu$L of the PGE$_2$ standard was put into tube 1 and mixed. Five-hundred microliter solution was taken from tube 1 and put into tube 2 and this process was repeated through tube 8.

Fifty microliters of EIA Buffer and 50$\mu$l DMEM were added into the NSB wells. Fifty $\mu$l DMEM was added to

the $B_0$ wells. Fifty microliters of solution was taken from tube #8 and added to both the lowest standard wells (S8). Fifty microliters was taken from tube #7 and added to each of the next two wells. Continue this through to tube #1. (Use the same pipet tip for all 8 of the standards. Make sure to equilibrate the tip in each new standard by pipeting up and down in that standard. Using a P200, add 50$\mu$l of each sample at each dilution to the sample wells).

Using the 12 channel pipetor, 50$\mu$l of the Prostaglandin $E_2$ acetylcholinesterase tracer was added to each well except the Total Activity (TA) and the Blank (B) wells. Using the 12 channel pipetor, 50$\mu$l of the Prostaglandin $E_2$ monoclonal antibody was added to each well except the Total Activity (TA), the (NSB), and the Blank (B) wells. The plate was covered with plastic film (item #7) and incubated for 18 hours at 4°C.

The plate was developed as follows: one 100$\mu$L vial of Ellman's Reagent (vial #8) was reconstituted with 50 ml of Ultra-pure water in a 50 mL centrifuge tube. It was protected from light and used the same day. The wells were and rinsed five times with Wash Buffer using a 12 channel pipettor. Two-hundred microliters of Ellman's Reagent was added to each well using a 12 channel pipettor and 5$\mu$l of Tracer to the (TA) well was then added to each well using a P10. The plate was covered with a plastic film and placed on orbital shaker in the dark for 60-90 minutes.

The plate was read in the Bio-tek plate reader at a single wavelength between 405 and 420 nm. Before reading each plate, the bottom was wiped with a Kim wipe. The plate should be read when the absorbance of the wells is in the range of 0.3-0.8 A.U. If the absorbance of the wells exceeds 1.5, wash and add fresh Ellmans' Reagent and redevelop.

Determination of Medium Inhibitory Concentration ($IC_{50}$) - The medium inhibitory concentration of the $CO_2$ hops extract for both COX-2 and COX-1 were assessed using CalcuSyn (BIOSOFT, biosoft.com). This statistical package performs multiple drug dose-effect calculations using the Median Effect methods described by T-C Chou and P. Talaly (Trends Pharmacol. Sci. 4:450-454). Briefly, it correlates the "Dose" and the "Effect" in the simplest possible form: fa/fu = $(C/Cm)^m$, where C is the concentration or dose of the compound and Cm is the median-effective dose signifying the potency. Cm is determined from the x-intercept of the median-effect plot. The fraction affected by the concentration of the test material is fa and the fraction unaffected by the concentration is fu (fu = 1 - fa). The exponent m is the parameter signifying the sigmoidicity or shape of the dose-effect curve. It is estimated by the slope of the median-effect plot.

The median-effect plot is a plot of x = log(C) vs y = log(fa/fu) and is based on the logarithmic form of Chou's median-effect equation. The goodness of fit for the data to the median-effect equation is represented by the linear correlation coefficient r of the median-effect plot. Usually, the experimental data from enzyme or receptor systems have r > 0.96, from tissue culture or enzyme work.

Results - The medium inhibitory concentration of COX-2 inhibition by the $CO_2$-extract of hops in the RAW 264.7 cell model was 0.24 $\mu$g/mL (95% CI = 0.16-0.36). The same $CO_2$ extract of hops demonstrated a median inhibitory concentration of COX-1 production of PGE2 of 25.5 $\mu$g/mL. Thus, a COX-1/COX-2 specificity of 106 is observed. This COX-2 specificity is 2.7-fold greater than the COX-2 specificity demonstrated for pure humulone in the TNFalpha stimulation of MC3T3-E1 cells [Yamamoto, K. 2000. Suppression of cyclooxygenase-2 gene transcription by humulon of bec hop extract studied with reference to glucocorticoid. FEBS Letters 465:103-106]. Such a large difference in COX-2 specificity between the pure compound and the complex mixture is unexpected and constitutes a novel finding. It is unusual that a complex mixture would contain greater specific biological activity than the most active molecule. The inference is that an underlying synergy among the bioactive molecules, including humulone, is to account for such an effect.

EXAMPLE2 2

Synergistic Inhibition of Prostaglandin E2 Production in Murine B Cells by Curcuminoids and an Extract of Hops

This example illustrates the superior COX-2 inhibitory potency and selectivity of the combination of curcuminoids and hops extract of the present invention compared to curcuminoids alone.

Inhibition of COX-2 Mediated Production of PGE2 in RAW 264.7 Cells - **Equipment -** balancer, analytical, Ohaus Explorer (Ohaus Model #EO1140, Switzerland), biosafety cabinet (Forma Model #F1214, Marietta, Ohio), pipettor, 100 to 1000 $\mu$L (VWR Catalog #4000-208, Rochester, NY), cell hand tally counter (VWR Catalog #23609-102, Rochester, NY), $CO_2$ incubator (Forma Model #F3210, Marietta, Ohio), hemacytometer (Hausser Model #1492, Horsham, PA), microscope, inverted (Leica Model #DM 1L, Wetzlar, Germany), multichannel pipettor, 12-Channel (VWR Catalog #53501-662, Rochester, NY), Pipet Aid (VWR Catalog #53498-103, Rochester, NY), Pipettor, 0.5 to 10 $\mu$L (VWR Catalog #4000-200, Rochester, NY), pipettor, 100 to 1000 $\mu$L (VWR Catalog #4000-208, Rochester, NY), pipettor, 2 to 20 $\mu$L (VWR Catalog #4000-202, Rochester, NY), pipettor, 20 to 200 $\mu$L (VWR Catalog #4000-204, Rochester, NY), PURELAB Plus Water Polishing System (U.S. Filter, Lowell, MA), refrigerator, 4°C (Forma Model #F3775, Marietta, Ohio), vortex mixer (VWR Catalog #33994-306, Rochester, NY), water bath (Shel Lab Model #1203, Cornelius, OR).

**Cells, Chemicals, Reagents and Buffers -** Cell scrapers (Corning Catalog #3008, Corning, NY), dimethylsulfoxide (DMSO) (VWR Catalog #5507, Rochester, NY), Dulbecco's Modification of Eagle's Medium (DMEM) (Mediatech Catalog #10-013-CV, Herndon, VA), fetal bovine serum, heat inactivated (FBS-HI) (Mediatech Catalog #35-011-CV, Herndon, VA), lipopolysaccharide (LPS)(Sigma Catalog #L-2654, St. Louis, MO), microfuge tubes, 1.7 mL (VWR Catalog #20172-698, Rochester, NY), penicillin/streptomycin (Mediatech Catalog #30-001-CI, Herndon, VA), pipet tips for 0.5 to 10 $\mu$L pipettor (VWR Catolog #53509-138, Rochester, NY), pipet tips for 100-1000 $\mu$L pipettor (VWR Catolog #53512-294, Rochester, NY), pipet tips for 2-20 $\mu$L and 20-200 $\mu$L pipettors (VWR Catolog #53512-260, Rochester, NY), pipets, 10 mL (Becton Dickinson Catalog #7551, Marietta, OH), pipets, 2 mL (Becton Dickinson Catalog #7507, Marietta, OH, pipets, 5 mL (Becton Dickinson Catalog #7543, Marietta, OH), RAW 264.7 Cells (American Type Culture Collection Catalog #TIB-71, Manassas, VA), test compounds (liquid $CO_2$ hops extract from Hopunion, Yakima, WA), tissue culture plates, 96-well (Becton Dickinson Catalog #3075, Franklin Lanes, NJ), Ultra-pure water (Resistance =18 megaOhm-cm deionized water).

*General Procedure -* RAW 264.7 cells, obtained from ATCC, were grown in DMEM medium and maintained in log phase growth. The DMEM growth medium was made as follows: 50 mL of heat inactivated FBS and 5 mL of penicillin/streptomycin were added to a 500 mL bottle of DMEM and stored at 4°C. This was warmed to 37 °C in a water bath before use and for best results should be used within three months

On day one of the experiment, the log phase 264.7 cells were plated at 8 x10$^4$ cells per well in 0.2 mL growth medium per well in a 96-well tissue culture plate. After 6 to 8 hours post plating, 100 $\mu$L of growth medium from each well was removed and replaced with 100 $\mu$L fresh medium. A 1.0 mg/mL solution of LPS, which was used to induce the expression of COX-2 in the RAW 264.7 cells, was prepared by dissolving 1.0 mg of LPS in 1 my DMSO. It was mixed until dissolved and stored at 4°C. Immediately before use, it was thawed at room temperature or in a 37°C water bath.

On day two of the experiment, the test materials were prepared as 1000X stock in DMSO. For example, if the final concentration of the test material was to be 10 $\mu$g/mL, a 10 mg/mL stock was prepared by dissolving 10 mg of the test material in 1 mL of DMSO. Fresh test materials were prepared on day 2 of the experiment. In 1.7 mL microfuge tubes, 1 mL DMEM without FBS was added to obtain test concentrations of 0.05, 0.10, 0.5, and 1.0 $\mu$g/mL. 2 $\mu$L of the 1000X DMSO stock of the test material was added to the 1 mL of medium without FBS. The tube contained the final concentration of the test material was concentrated 2-fold. The tube was placed in incubator for 10 minutes to equilibrate.

One-hundred mL of medium was removed from each well of the cell plates prepared on day one. One-hundred mL of equilibrated 2X final concentration the test compounds were added to cells and incubated for 90 minutes. LPS in DMEM without FBS was prepared by adding 44 $\mu$L of the 1 mg/mL DMSO stock to 10 mL of medium. For each well of cells to be stimulated, 20 $\mu$L of LPS (final concentration of LPS is 0.4 $\mu$g/mL of LPS) was added. The LPS stimulation was continued for 24 hours, after which the supernatant medium from each well was transferred to a clean microfuge tube for determination of the PGE2 content in the medium.

Determination of COX-1 Enzyme Inhibition by Curcuminoids and hops extract - The ability of a test material to inhibit COX-1 synthesis of PGE2 was determined essentially as described by Noreen, Y., et al. (J. Nat. Prod. 61, 2-7, 1998).

*Equipment -* balancer (2400 g, Acculab VI-2400, V W R Catalog # 11237-300, Rochester, NY), balancer, analytical, Ohaus Explorer (Ohaus Model #EO1140, Switzerland), biosafety cabinet (Forma Model #F1214, Marietta, Ohio), Freezer, -30°C (Fonna Model #F3797), Freezer, -80°C Ultralow (Forma Model #F8516, Marietta, OH), heated stirring plate (VWR Catalog #33918-262, Rochester, NY), ice maker (Scotsman Model #AFE400A-1A, Fairfax, SC), multichannel pipettor, 12-Channel (VWR Catalog #53501-662, Rochester, NY), Multichannel Pipettor, 8-Channel (VWR Catalog #53501-660, Rochester, NY), orbital shaker platform (Scienceware #F37041-0000, Pequannock, NJ), pH meter (VWR Catalog #33221-010, Rochester, NY), pipet aid (VWR Catalog #53498-103, Rochester, NY), pipettor, 0.5 to 10 $\mu$L (VWR Catalog #4000-200, Rochester, NY), pipettor, 100 to 1000 $\mu$L (VWR Catalog #4000-208, Rochester, NY), pipettor, 2 to 20 $\mu$L (VWR Catalog #4000-202, Rochester, NY), pipettor, 20 to 200 $\mu$L (VWR Catalog #4000-204, Rochester, NY), PURELAB Plus Water Polishing System (U.S. Filter, Lowell, MA), refrigerator, 4°C (Fonna Model #F3775, Marietta, Ohio), vacuum chamber (Sigma Catalog #Z35, 407-4, St. Louis, MO), vortex mixer (VWR Catalog #33994-306, Rochester, NY)

*Supplies and Reagents -* 96-Well, round-bottom plate (Nalge Nunc #267245, Rochester, NY), arachidonic acid (Sigma Catalog #A-3925, St. Louis, MO), centrifuge tubes, 15 mL, conical, sterile (VWR Catalog #20171-008, Rochester, NY), COX-1 enzyme (ovine) 40,000 units/mg (Cayman Chemical Catalog #60100, Ann Arbor, M1), dimethylsulfoxide (DMSO) (VWR Catalog #5507, Rochester, NY), ethanol 100% (VWR Catalog #MK701908, Rochester, NY), epinephrine (Sigma Catalog #E-4250, St. Louis, MO), glutathione (reduced) (Sigma Catalog # G-6529, St. Louis, MO), graduated cylinder, 1000 mL (VWR Catalog #24711-364, Rochester, NY), hematin (porcine) (Sigma catalog # H-3281, St. Louis, MO), hydrochloric acid (HCl) (VWR Catalog #VW3110-3, Rochester, NY), KimWipes (Kimberly Clark Catalog #34256, Roswell, GA), microfuge tubes, 1.7 mL (VWR Catalog #20172-698, Rochester, NY), NaOH (Sigma Catalog #S-5881, St. Louis, MO), pipet tips for 0.5 to 10 $\mu$L pipettor (VWR Catolog #53509-138, Rochester, NY), pipet tips for 100-1000 $\mu$L pipettor (VWR Catolog #53512-294, Rochester, NY), pipet tips for 2-20 $\mu$L and 20-200 $\mu$L pipettors (VWR Catolog #53512-260, Rochester, NY), prostaglandin E2 (Sigma Catalog # P-5640, St. Louis, MO), prostaglandin F2alpha (Sigma Catalog # P-0424, St. Louis, MO), stir bar, magnetic (VWR Catalog #58948-193, Rochester, NY), storage bottle, 1000 mL (Coming Catalog #1395-1L, Corning, NY), storage bottle, 100 mL (Corning Catalog #1395-100, Corning, NY), $CO_2$ extract of hops (Hopunion, Yakima, WA), Tris-HCl (Sigma Catalog #T-5941, St. Louis, MO), ultra-pure water (Resistance = 18 megaOhm-cm deionized water).

*General Procedure* - Oxygen-free 1.0M Tris-HCl buffer (pH 8.0) was prepared as follows. In a 1000 mL beaker, 12.11g Trizma HCl was dissolved into 900 mL ultra-pure water. The beaker was placed on a stir plate with a stir bar. NaOH was added until the pH reached 8.0. The volume was adjusted to a final volume of 1000mL and stored in a 1000 mL storage bottle.

The Tris-HCl buffer was placed into a vacuum chamber with the top loosened and the air pump was turned on until the buffer stopped bubbling. The vacuum chamber was then turned off and the storage bottle was tightly covered. This step was repeated each time when oxygen-free Tris-HCl buffer was used.

One mL cofactor solution was prepared by adding 1.3 mg (-) epinephrine, 0.3 mg reduced glutathione and 1.3 mg hematin to 1 mL oxygen free Tris-HCl buffer. The solutions of the test material were prepared as needed. i.e. 10 mg of aspirin was weighed and dissolved into 1 mL DMSO.

Enzymes, i.e. prostaglandin E2 or prostaglandin F2alpha, were dissolved in oxygen free Tris-HCl buffer as follows, i.e. on ice, 6.5 μL of enzyme at 40,000 units/mL was taken and added to 643.5 μL of oxygen free Tris-HCl buffer. This enzyme solution is enough for 60 reactions. The COX-1 enzyme solution was prepared as follows: In a 15 mL centrifuge tube, 10 μL COX-1 enzyme at 40,000 units/mL was added to oxygen free Tris-HCl with 50 μL of the cofactor solution per reaction. The mixture was incubated on ice for 5 minutes. For 60 reactions, 650 μL enzyme were added in oxygen free Tris-HCl buffer with 3.25 mL cofactor solution.

Sixty microliters of the enzyme solution were combined with 20 μL of the test solution in each well of a 96 well plate. Final concentrations of the test solutions were 100, 50, 25, 12.5, 6.25 and 3.12 μg/mL. The plates were preincubated on ice for 10 minutes. Twenty μL arachidonic acid (30μM) was added and incubated for 15 minutes at 37°C.

Two M HCl was prepared by diluting 12.1 N HCl in a 100 mL storage bottle. 83.5 mL ultra-pure water was added and then 16.5 mL 12.1 N HCl was added. It was stored in a 100 mL storage bottle and placed in the Biosafty cabinet. The reaction was terminated by adding 10 μL 2 M HCl. The final solution was used as the supernatant for the PGE2 assay.

Determination of PGE2 Concentration in Medium - The procedure followed was that essentially described by Hamberg, M. and Samuelsson, B. (J. Biol. Chem. 1971. 246, 6713-6721); however a commercial, nonradioactive procedure was employed.

*Equipment* - freezer, -30°C (Forma Model #F3797), heated stirring plate (VWR Catalog #33918-262, Rochester, NY), multichannel pipettor, 12-Channel (VWR Catalog #53501-662, Rochester, NY), orbital shaker platform (Scienceware #F37041-0000, Pequannock, NJ), Pipet Aid (VWR Catalog #53498-103, Rochester, NY), pipettor, 0.5 to 10 μL (VWR Catalog #4000-200, Rochester, NY), pipettor, 100 to 1000 μL (VWR Catalog #4000-208, Rochester, NY), pipettor, 2 to 20 μL (VWR Catalog #4000-202, Rochester, NY), pipettor, 20 to 200 μL (VWR Catalog #4000-204, Rochester, NY), plate reader (Bio-tek Instruments Model #E1x800, Winooski, VT), PURELAB Plus Water Polishing System (U.S. Filter, Lowell, MA), refrigerator, 4°C (Forma Model #F3775, Marietta, Ohio).

*Chemicals, Reagents and Buffers* - Prostaglandin E2 EIA Kit-Monoclonal 480-well (Cayman Chemical Catalog # 514010, Ann Arbor, MI), centrifuge tube, 50 mL, conical, sterile (VWR Catalog #20171-178, Rochester, NY), Dulbecco's Modification of Eagle's Medium (DMEM) (Mediatech Catalog #10-013-CV, Herndon, VA), graduated cylinder, 100 mL (VWR Catalog #24711-310, Rochester, NY), KimWipes (Kimberly Clark Catalog #34256, Roswell, GA), microfuge tubes, 1.7 mL (VWR Catalog #20172-698, Rochester, NY), penicillin/streptomycin (Mediatech Catalog #30-001-C1, Herndon, VA), pipet tips for 0.5 to 10 μL pipettor (VWR Catolog #53509-138, Rochester, NY), pipet tips for 100-1000 μL pipettor (VWR Catolog #53512-294, Rochester, NY), pipet tips for 2-20 μL and 20-200 μL pipettors (VWR Catolog #53512-260, Rochester, NY), pipets, 25 mL (Becton Dickinson Catalog #7551, Marietta, OH), storage bottle, 100 mL (Corning Catalog #1395-100, Corning, NY), storage bottle, 1000 mL (Coming Catalog #1395-1L, Corning, NY), ultra-pure water (Resistance = 18 megaOhm-cm deionized water).

*General Procedure* - EIA Buffer was prepared by diluting the contents of the EIA Buffer Concentrate (vial #4) with 90ml of Ultra-pure water. Vial #4 was rinsed several times to ensure all crystals had been removed and was then placed into a 100 mL storage bottle and stored at 4°C.

The Wash Buffer was prepared by diluting Wash Buffer Concentrate (vial #5) 1:400 with Ultra-pure water. 0.5 ml/liter of Tween 20 (vial #5a) was then added (using a syringe for accurate measurement). To prepare one liter of Wash Buffer add 2.5ml Wash Buffer Concentrate, 0.5ml Tween-20, and 997ml Ultra-pure water. The solution was stored in a 1 liter storage bottle at 4°C.

The Prostaglandin E2 standard was reconstituted as follows. A 200μL pipet tip was equilibrated by repeatedly filling and expelling the tip several times in ethanol. The tip was used to transfer 100 μL of the PGE2 Standard (vial #3) into a 1.7 mL microfuge tube. 900μl Ultra-pure water was added to the tube and stored at 4°C, which was stable for -6 weeks. The Prostaglandin E2 acetylcholinesterase tracer was reconstituted as follows. 100 μL PGE2 tracer (vial #2) was mixed with 30 mL of the EIA Buffer in a 50 mL centrifuge tube and stored at 4°C.

The Prostaglandin E2 monoclonal antibody was reconstituted as follows. 100μL PGE2 Antibody (vial #1) was mixed with 30 mL of the EIA buffer in a 50 mL centrifuge tube and stored at 4°C.

DMEM with penicillin/streptomycin was prepared by adding 5 mL penicillin/streptomycin into 500 mL DMEM and stored at 4°C

The plates were set up as follows: Each plate contained a minimum of two blanks (B), two non-specific binding wells (NSB), two maximum binding wells ($B_0$), and an eight point standard curve run in duplicate (S1-S8). Each sample was assayed at a minimum of two dilutions and each dilution was run in duplicate.

The standard was prepared as follows: Eight 1.7 mL microfuge tubes were labeled as tubes 1-8. 900 $\mu$L DMEM into was added to tube 1 and 500 $\mu$L DMEM to tubes 2-8. 100 $\mu$L of the PGE2 standard was added to tube I and mixed. Five-hundred mL of solution was taken from tube 1 and put into tube 2, and this process was repeated through tube 8.

Fifty mL EIA Buffer and 50$\mu$l DMEM were added into the NSB wells. Fifty $\mu$l DMEM was added to the $B_0$ wells. Fifty mL of solution was taken from tube #8 and added to both the lowest standard wells (S8). Fifty mL was taken from tube #7 and added to each of the next two wells. This was continued through to tube #1. The same pipet tip was used for all 8 of the standards making sure to equilibrate the tip in each new standard by pipeting up and down in that standard. Using a P200, 50$\mu$l of each sample at each dilution was added to the sample wells.

Using a 12 channel pipetor, 50$\mu$l of the Prostaglandin E2 acetylcholinesterase tracer was added to each well except the Total Activity (TA) and the Blank (B) wells. Using the 12 channel pipetor, 50$\mu$l of the Prostaglandin E2 monoclonal antibody was added to each well except the Total Activity (TA), the (NSB), and the Blank (B) wells. The plate was covered with plastic film (item #7) and incubated for 18 hours at 4°C.

The plates were developed as follows: one 100 $\mu$L vial of Ellman's Reagent (vial #8) was reconstituted with 50 ml of Ultra-pure water in a 50 mL centrifuge tube. It was protected from light and used the same day. The wells were washed and rinsed five times with Wash Buffer using a 12 channel pipettor. Two-hundred mL of Ellman's Reagent was added to each well using a 12 channel pipettor and 5$\mu$l of Tracer to the total activity (TA) wells was then added to each well using a P10 pipette. The plate was covered with a plastic film and placed on orbital shaker in the dark for 60-90 minutes.

The plate was read in the Bio-tek plate reader at a single wavelength between 405 and 420 nm. Before reading each plate, the bottom was wiped with a Kim wipe. The plate should be read when the absorbance of the wells is in the range of 0.3-0.8 A.U. If the absorbance of the wells exceeded 1.5, they were washed and fresh Ellmans Reagent was added and then redeveloped.

Calculation of synergy and combination index - Synergy between the curcuminoids and andrographolide was assessed using CalcuSyn (BIOSOFT, biosoft.com). This statistical package performs multiple drug dose-effect calculations using the Median Effect methods described by T-C Chou and P. Talaly (Trends Pharmacol. Sci. 4:450-454), hereby incorporated by reference.

Briefly, it correlates the "Dose" and the "Effect" in the simplest possible form: fa/fu = (C/Cm)m, where C is the concentration or dose of the compound and Cm is the median-effective dose signifying the potency. Cm is determined from the x-intercept of the median-effect plot. The fraction affected by the concentration of the test material is fa and the fraction unaffected by the concentration is fu (fu = 1 - fa). The exponent m is the parameter signifying the sigmoidicity or shape of the dose-effect curve. It is estimated by the slope of the median-effect plot.

The median-effect plot is a plot of x= log(C) vs y = log(fa/fu) and is based on the logarithmic form of Chou's median-effect equation. The goodness of fit for the data to the median-effect equation is represented by the linear correlation coefficient r of the median-effect plot. Usually, the experimental data from enzyme or receptor systems have an r > 0.96, from tissue culture an r > 0.90 and from animal systems an r > 0.85.

Synergy of test components is quantified using the combination index (CI) parameter. The CI of Chou-Talaly is based on the multiple drug-effect and is derived from enzyme kinetic models (Chou, T.-C. and Talalay, P. (1977). A simple generalized equation for the analysis of multiple inhibitions of Michaelis-Menten kinetic systems. J. Biol. Chem. 252:6438-6442). The equation determines only the additive effect rather than synergism or antagonism. However, we define synergism as a more than expected additive effect, and antagonism as a less than expected additive effect as proposed by Cho and Talalay in 1983 (Trends Pharmacol. Sci. (1983) 4:450-454). Using the designation of C1 = 1 as the additive effect, we obtain for mutually exclusive compounds that have the same mode of action or for mutually non-exclusive drugs that have totally independent modes of action the following relationships: CI < 1, = 1, and > 1 indicating synergism, additivity and antagonism, respectively.

Expected median inhibitory concentrations of the two-component combinations were estimated using the relationship:

$$[1/\text{Expected IC}_{50}] = [A/\text{IC}_{50}A] + [B/\text{IC}_{50}B]$$

where A = mole fraction of component A in the combination and B = the mole fraction of component B in the combination.

The observed and expected median inhibitory concentrations for curcumin and hops extract for PGE2 production by COX-2 in the RAW 264.7 cell assay were determined. While the expected $IC_{50}$ for the 10:1 combination of curcum and hops extract was 1.6 $\mu$g/mL, the observed value was 0.77 $\mu$g/mL or 2-fold greater. This level of difference was unexpected and constitutes a novel finding for the combined COX-2 inhibitory activity of the 1:10 combination of curcumin and hops extract.

Statistical analysis of inhibition of COX-2 production of PGE2 in the RAW 264.7 cell model for the 1:10 combination of curcumin and hops extract was determined. The CI for this combination was 0.490, 0.472 and 0.454, respectively, for the $IC_{50}$, $IC_{75}$ and $IC_{90}$. These CI values indicate strong synergy between curcumin and hops extract over the complete dose-response curve.

The medium inhibitory concentration of COX-2 by curcumin alone in the RAW 264.7 cell model was 4.01 $\mu$g/mL. Inhibition of COX-1 enzyme activity by curcumin was somewhat higher with an $IC_{50}$ of 10.0 $\mu$g/mL. Hops extract exhibited an $IC_{50}$ of PGE2 inhibition by COX-2 of 0.21 $\mu$g/mL and an $IC_{50}$ for COX-1 enzyme inhibition estimated at 6.25 $\mu$g/mL; the COX-2 specificity of curcumin alone was 2.5 and for hops extract, it was 29.5. Eleven formulations of curcumin and hops extract exhibited COX-2 specificity ranging from 48.6 to 11.2, with a median COX-2 specificity of 17.4. All of the combinations of curcumin and hops extract unexpectedly demonstrated COX-2 specificity greater than the nominal 5.0 suggested as the minimum for pharmaceutical products designed to limit PGE2 production specifically through inhibition of COX-2. This finding indicates that combinations of curcumin and a hops extract could function as potent anti-inflammatory formulations without the GI side effects seen with COX-1 inhibition.

EXAMPLE 3

Normalization of Joint Functioning Following Trauma

A representative composition of the present invention as a dietary supplement would be in an oral formulation, i.e. tablets, that would supply one of the following combinations: (a) 15 mg curcuminoid/kg per day and 6.0 mg humulone/kg per day; (b) 15 mg curcuminoid/kg per day and 6.0 mg upulons/kg per day; (c) 15 mg curcuminoid/kg per day and 6.0 mg dihydroisohumulones/kg per day. Normalization of joint movement following physical trauma due to exercise or repetitive movement stress would be expected to occur following two to ten doses. This result would be expected in all animals.

EXAMPLE 4

Clinical Effectiveness of Lotion Formulations in the Treatment of Acne Rosacea

A lotion designed to contain one of the following: (a) 0.1% wt curcuminoids and 0.5% humulone; or (b) 0.1% wt curcuminoids and 0.5% lumulone is applied to affected areas of patients who have exhibited acne rosace as diagnosed by their health practitioner and confirmed by an independent board-certified dermatologist. Self-evaluation tests are administered one week prior to the study to quantify the surface area affected and redness. In addition, similar variables are scored by the professional clinical staff not aware of the patients treatment status. These evaluations are repeated on Days 0, 7, 14 and 21.

Patients are randomly assigned to the test formulation or placebo at the start of the study. The test formulation and placebo are applied to the affected area one or two times per day. Treatment for health conditions such as diabetes, hypertension, etc. is allowed during the study. Scores are statistically compared between the test formulation and the placebo for each of the four observational periods. Patients treated with the composition of the present invention in a lotion formulation are considered improved if the patients' scores improve by greater than 20% from the pre-test scores within each category evaluated. The percentage of persons exhibiting improvement is compared between the combination formulations and the placebo control. The difference between the two groups is considered statistically significant if the probability of rejecting the null hypothesis when true is less than five percent.

EXAMPLE 5

Clinical Effectiveness of Lotion Formulation in the Treatment of Psoriasis

This example is performed in the same manner as described in Example 4, except that the composition is applied to affected areas of patients who have exhibited psoriasis as diagnosed by their own practitioner and confirmed by an independent board-certified dermatologist. Self-evaluation tests are administered one week prior to the study to quantify the surface area affected and skin condition. In addition, similar variables are scored by the professional clinical staff not aware of the patients treatment status. These evaluations are repeated on Days 0, 7, 30 and 60.

Patients are randomly assigned to the test formulation or placebo at the start of the study. The test formulation and placebo are applied to the affected area one or two times per day. Treatment for health conditions such as diabetes, hypertension, etc. is allowed during the study. Scores are statistically compared between the test formulation and the placebo for each of the four observational periods. Patients treated with the composition of the present invention as the test lotion formulation are considered improved if the patients' scores improve by greater than 20% from the pre-test scores within each category evaluated. The percentage of persons exhibiting improvement is compared between the test formulation and the placebo control. The difference between the two groups is considered statistically significant if the probability of rejecting the null hypothesis when true is less than five percent.

EXAMPLE 6

Clinical Effectiveness of a Formulation in the Treatment of Alzheimer's Disease

An oral formulation as described in Example 3 is administered to patients who have manifested an early stage of Alzheimer's Disease (AD), as diagnosed by their practitioner and confirmed by an independent board-certified neurologist. Two weeks before the clinical trial, the patients undergo appropriate psychoneurological tests such as the Mini Mental Status Exam (MMSE), the Alzheimer Disease Assessment Scale (ADAS), the Boston Naming Test (BNT), and the Token Test (TT). Neuropsychological tests are repeated on Day 0, 6 weeks and 3 months of the clinical trial. The tests are performed by neuropsychologists who are not aware of the patient's treatment regimen.
Patients are randomly assigned to the test formulation or placebo at the start of the study. The test formulation and placebo are taken orally one or two times per day. Treatment for conditions such as diabetes, hypertension, etc. is allowed during the study. Scores are statistically compared between the test formulation and the placebo for each of the three observational periods. Without treatment, the natural course of AD is significant deterioration in the test scores during the course of the clinical trial. Patients treated with the composition of the present invention as the test formulation are considered improved if the patients' scores remain the same or improve during the course of the clinical trial.

EXAMPLE 7

Oral Formulation in the Treatment and Prevention of Colon Cancer

An oral formulation as described in Example 3 is administered to patients who have manifested an early stage of colon cancer as diagnosed by their own practitioner and confirmed by an independent board-certified oncologist.
Patients are randomly assigned to the test formulation or a placebo at the start of the study. The test formulation and placebo are taken orally one or two times per day. Treatment for conditions such as diabetes, hypertension, etc. is allowed during the study. Endoscopic evaluations are made at one, two, six and twelve months. Evidence of reappearance of the tumor during any one of the four follow-up clinical visits is considered a treatment failure. The percentage of treatment failures is compared between the test formulation and the placebo control. Under the experimental conditions described, the test material is expected to decrease the tumor incidence with respect to the control group. The difference between the two groups is considered statistically significant if the probability of rejecting the null hypothesis when true is less than five percent.

EXAMPLE 8

Oral Formulation for the Treatment of Irritable Bowel Syndrome

An oral formulation as described in Example 3 is administered to patients who have manifested irritable bowel syndrome as diagnosed by their practitioner. Normal bowel functioning is restored within 24 hours.

EXAMPLE 9

Normalization of Joint Functioning in Osteoarthritis

Using compositions described in Example 3 normalization of joint stiffness due to osteoarthritis occurs following five to twenty doses, in the presence or absence of glucosamine or chondroitin sulfate. In addition, the composition does not interfere with the normal joint rebuilding effects of these two proteoglycan constituents, unlike traditional non-steroidal anti-inflammatory agents.
In summary, one embodiment of the present invention is a composition for inhibition of inducible COX-2 activity and having minimal effect on COX-1 activity, said composition comprising, as a first component an effective amount of a curcuminoid species and an effective amount of a second component selected from the group consisting of an alpha-acid species and a beta-acid species or derivatives thereof. The curcuminoid species is curcumin, demethoxycurcurmin, or bisdemethoxycurcumin. the alpha-acid species is preferably humulone, cohumulone, isohumulone, isoprehumulone, hulupone, adhumulone, xanthohumol A or xanthohumol B. The beta-acid species is preferably lupulone, colupulone, adlupulone, tetrahydroisohuniulone, hexahydrocolupulone or dihydro-isohumulone. The first or the second components of the present composition may be of pharmaceutical grade or derived from plant(s) or plant extract(s). The first or second components may also be conjugated with a compounds such as mono- or di- saccharides, amino acids, sulfates, succinates, acetates or glutathione. The compositions of the present invention may be formulated in a pharmaceutically acceptable carrier and contain additives such as antioxidants, vitamins, minerals proteins, fats, carbohydrates, glucosamine, chondrotin sulfate or aminosugars.

Other embodiments of the present invention includes methods of dietary supplementation of the compositions of the present invention to reduce the symptoms in animals suffering from symptoms of inflammation. The composition is formulated in a dosage form such that said administration provides from 0.001 to 30.0 mg body weight per day of each curcuminoid species, and from 0.5 to 20.0 mg/kg bodyweight per day of alpha-acid species or beta-acid species. The composition is administered in an amount sufficient to maintain a serum concentration of 0.1 to 50 $\mu$M of each curcuminoid species, and from 0.001 to 50 $\mu$M of each alpha-acid species or beta-acid species. The animal may be humans, non-human primates, dogs, cats, birds, reptiles, amphibians, horses or ruminants. The administration may be an oral, parenteral, topical, transdermal or transmucosal delivery system.

Thus, among the various formulations taught there has been disclosed a formulation comprising curcuminoids, as the first component, and a second compound selected from the group consisting of alpha-acids and beta-acids. These combinations provide for a synergistic anti-inflammatory effect in response to physical or chemical injury or abnormal immune stimulation due to a biological agent or unknown etiology. It will be readily apparent to those skilled in the art that various changes and modifications of an obvious nature may be made without departing from the spirit of the invention, and all such changes and modifications are considered to fall within the scope of the invention as defined by the appended claims. Such changes and modifications would include, but not be limited to, the incipient ingredients added to affect the capsule, tablet, lotion, food or bar manufacturing process as well as vitamins, herbs, flavorings and carriers. Other such changes or modifications would include the use of other herbs or botanical products containing the combinations of the present invention disclosed above.

**Claims**

1. The use of a first component selected from a curcuminoid and a second component selected from an alpha acid and a beta acid in the manufacture of a medicament for the treatment of diabetes.

2. The use of claim 1 , wherein said curcuminoid is selected from curcumin, demethoxycurcumin, and bisdemethoxycurcumin.

3. The use of claim 1 , wherein the alpha acids are selected from the group consisting of humulone, cohumulone, isohumulone, isoprehumulone, hulupone, adhumulone, xanthohumol A and xanthohumol B.

4. The use of claim 1 , wherein the beta acids are selected from the group consisting of lupulone, colupulone, adlupulone, tetrahydroisohumulone, hexahydrocolupulone, and dihydro-isohumulone.

5. The use of claim 1 , wherein said composition is formulated in a pharmaceutically acceptable carrier.

6. The use of claim 1 , wherein said composition further comprises one or more members selected from the group consisting of antioxidants, vitamins and minerals.

7. The use of claim 1 , wherein said composition further comprises one or more members selected from the group consisting of proteins, fats, carbohydrates, glucosamine, chondrotin sulfate and aminosugars.

8. The use of claim 1 , wherein said first or second component is conjugated with monosaccharides, disaccharides, amino acids, sulfates, succinates, acetates or glutathione.

[A]

[B]

[C]

[D]

[E]

[F]

Fig. 1

[A]

[B]

or

Fig. 2

[A]

[B]

Fig. 3

Europäisches
Patentamt

European
Patent Office

Office européen
des brevets

# EUROPEAN SEARCH REPORT

Application Number

EP 10 00 6768

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2005/084230 A2 (METAPROTEOMICS LLC [US]; BABISH JOHN G [US]; TRIPP MATTHEW L [US]; BLA) 15 September 2005 (2005-09-15) * claims 16,21,22 * * page 21, paragraph 076 * ----- | 1-5 | INV. A61K31/12 A61K36/9066 A61P3/10 |
| X | WO 03/035007 A (METAPROTEOMICS LLC [US]) 1 May 2003 (2003-05-01) * claims 1-25 * * page 10, line 13 * * table 3 * ----- | 1-8 | |
| A | WO 03/000185 A2 (METAPROTEOMICS LLC [US]; BABISH JOHN G [US]; HOWELL M TERRENCE [US]) 3 January 2003 (2003-01-03) * the whole document * ----- | 1-8 | |

TECHNICAL FIELDS
SEARCHED      (IPC)

A61K

The present search report has been drawn up for all claims

| Place of search | Date of completion of the search | Examiner |
|---|---|---|
| Munich | 28 October 2010 | Baurand, Petra |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
........................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 10 00 6768

This annex lists the patent family members relating to the patent documents cited in the above-mentioned European search report.
The members are as contained in the European Patent Office EDP file on
The European Patent Office is in no way liable for these particulars which are merely given for the purpose of information.

28-10-2010

| Patent document cited in search report | | Publication date | Patent family member(s) | | Publication date |
|---|---|---|---|---|---|
| WO 2005084230 | A2 | 15-09-2005 | AU | 2005218319 A1 | 15-09-2005 |
| | | | CA | 2557643 A1 | 15-09-2005 |
| | | | CN | 1946409 A | 11-04-2007 |
| | | | CN | 101690744 A | 07-04-2010 |
| | | | EP | 1718312 A2 | 08-11-2006 |
| | | | JP | 2007525523 T | 06-09-2007 |
| | | | US | 2007185213 A1 | 09-08-2007 |
| | | | US | 2005191375 A1 | 01-09-2005 |
| WO 03035007 | A | 01-05-2003 | AT | 482696 T | 15-10-2010 |
| | | | AU | 2002348096 B2 | 03-04-2008 |
| | | | AU | 2008202916 A1 | 24-07-2008 |
| | | | CA | 2464334 A1 | 01-05-2003 |
| | | | EP | 1446136 A2 | 18-08-2004 |
| | | | JP | 2005506996 T | 10-03-2005 |
| | | | MX | PA04003734 A | 20-06-2005 |
| | | | NZ | 532560 A | 25-02-2005 |
| WO 03000185 | A2 | 03-01-2003 | CA | 2450478 A1 | 03-01-2003 |
| | | | EP | 1423132 A2 | 02-06-2004 |
| | | | JP | 2004534806 T | 18-11-2004 |
| | | | JP | 2009203244 A | 10-09-2009 |
| | | | NZ | 530213 A | 27-01-2006 |
| | | | US | 2003113393 A1 | 19-06-2003 |
| | | | US | 2007172532 A1 | 26-07-2007 |
| | | | US | 2003008021 A1 | 09-01-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 09885721 B
- US 10282236 B

- US 60335062 B


### Non-patent literature cited in the description

- **Tobe, H. et al.** Bone resorption Inhibitors from hop extract. *Biosci. Biotech. Biochem,* 1997, vol. 61 (1), 158-159
- **Yamamoto, K.** Suppression of cyclooxygenase-2 gene transcription by humulon of bee hop extract studied with reference to glucocorticoid. *FEBS Letters,* 2000, vol. 465, 103-106
- **Lopes Vaz, A.** Double-blind clinical evaluation of the relative efficacy of ibuprofen and glucosamine sulphate in the management of osteoarthritis of the knee in outpatients. *Curr. Med Res Opin.,* 1982, vol. 8, 145-149
- **Noreen, Y. et al.** *J. Nat. Prod.,* 1998, vol. 61, 2-7

- **Hamberg, M. ; Samuelsson, B.** *J. Biol. Chem.,* 1971, vol. 246, 6713-6721
- **T-C Chou ; P. Talaly.** *Trends Pharmacol. Sci.,* vol. 4, 450-454
- **Yamamoto, K.** Suppression of cyclooxygenase-2 gene transcription by humulon of bec hop extract studied with reference to glucocorticoid. *FEBS Letters,* 2000, vol. 465, 103-106
- **Chou, T.-C. ; Talalay, P.** A simple generalized equation for the analysis of multiple inhibitions of Michaelis-Menten kinetic systems. *J. Biol. Chem.,* 1977, vol. 252, 6438-6442
- **Cho ; Talalay.** *Trends Pharmacol. Sci.,* 1983, vol. 4, 450-454